# EUROPEAN PATENT SPECIFICATION

(11) **EP 4 178 655 B1**
(45) Date of publication and mention of the grant of the patent: **26.03.2025**
(21) Application number: 21739094.7
(22) Date of filing: 01.07.2021
(51) Int. Cl.: A61M 25/00, A61M 25/01, C08G 75/045, C08G 18/38, C08G 18/83, C08L 75/14, C08K 3/38, A61L 29/06, A61L 29/14

(54) **USE OF A THERMOSET SHAPE MEMORY MATERIAL IN A THREAD-LIKE VARIABLE STIFFNESS DEVICE AND THREAD-LIKE VARIABLE STIFFNESS DEVICE**
VERWENDUNG EINES DUROPLASTISCHEN FORMGEDÄCHTNISMATERIALS IN EINER FADENARTIGEN VORRICHTUNG MIT VARIABLER STEIFIGKEIT UND FADENARTIGE VORRICHTUNG MIT VARIABLER STEIFIGKEIT
UTILISATION D'UN MATÉRIAU THERMODURCISSABLE À MÉMOIRE DE FORME DANS UN DISPOSITIF FILIFORME À RIGIDITÉ VARIABLE ET DISPOSITIF FILIFORME À RIGIDITÉ VARIABLE

(30) Priority: 09.07.2020 EP 20184854
(43) Date of publication of application: 17.05.2023
(73) Proprietor: ETH Zurich, 8092 Zürich (CH)
(72) Inventor: DE MARCO, Carmela, 5000 Aarau (CH); MATTMANN, Michael, 8046 Zürich (CH); LUSSI, Jonas, 8037 Zürich (CH); CHAUTEMS, Christophe, 8006 Zürich (CH); NELSON, Bradley James, 8126 Zumikon (CH)
(74) Representative: Schaad, Balass, Menzl & Partner AG
(86) International application number: PCT/EP2021/068122
(87) International publication number: WO 2022/008330

(56) References cited:
- WO-A1-2016/126703
- US-A- 5 662 621
- US-A1- 2007 112 331
- US-A1- 2016 193 810

## Description

The present invention relates to a thread-like variable stiffness device having improved flexibility to navigate tortuous conduits, in particular a catheter to navigate tortuous vessels.

A plastic material is any of a wide range of synthetic or semi-synthetic organic solids that are moldable. Plastics are typically organic polymers of high molecular mass, but they often contain other substances.

There are two types of plastics: thermoplastic polymers and thermosetting polymers. Thermoplastic polymers are the plastics that do not undergo chemical change in their composition when heated and can be molded again and again. In contrast, thermosetting polymers can melt and take shape once; after they have solidified, they stay solid. In the thermosetting process, a chemical reaction occurs that is irreversible.

US2008009831A1 discloses a catheter assembly including an elongate shaft comprising a thermoplastic polymer such as a thermoplastic shape memory polymer having a pre-selected glass transition temperature (Tg) and a means for heating the thermoplastic polymer, wherein the thermoplastic polymer is in a rubbery state at temperatures above the glass transition temperature and is in a glassy state at temperatures below the glass transition temperature. The elongate shaft may be selectively heated and cooled to provide sufficient flexibility and retention during a medical procedure. However, such a catheter comprises typically a guidewire or another device arranged in the catheter and subjected to thermal energy to heat in turn the thermoplastic polymer. Further, the catheter typically comprises an outer layer disposed about at least a portion of a thermoplastic polymer layer and needed to increase the flexibility of the elongate shaft to a desired level. This is a significant drawback when the diameter of the device must be reduced to produce minimally invasive surgical devices.

EP3449965A1 discloses a steerable catheter comprising portions of different stiffness based on a phase change between a liquid phase and a solid phase of a liquid-solid material. The liquid-solid material acts as a variable stiffness material and is contained in a double walled catheter providing a cavity for this variable stiffness material. Further, the catheter has additional control elements configured to influence the phase of the variable stiffness material. The use of low melting point alloys as variable stiffness material is disclosed. Such a material is potentially toxic and must be encapsulated in the cavity provided in the double walled catheter. The provision of this cavity along the catheter significantly limits the reduction of the diameter of the catheter. Further, in case of accidental rupture of the encapsulation, there is a risk of leakage of toxic substances in the body.

US 5 662 621 A1 discloses a catheter assembly including a generally elongate shaft formed of shaped memory material having a proximal end and a distal end. A lumen extends longitudinally between the proximal end and the distal end. The catheter assembly includes a core member insertable within the lumen for selectively providing a stimulus to at least a portion of the catheter shaft. The portion of the shaft is formed of a material responsive to the stimulus for changing between a first ductile state and a second, relatively more stiff, curved state. The material responsive to the stimulus may be a thermoset polymer such as a thermoset polyethylene. The core member may further comprise at least one heating member carried by the shaft and a control mechanism for selectively controlling the heating member. A mechanism may be included for selectively controlling a distal portion of the shaft while the shaft is in a ductile state.

US 2007/112331 A1 discloses a catheter including or being formed from a micromachined hypotube. The hypotube has a proximal region and a distal region. A number of slots are disposed along the length of the hypotube to confer flexibility. An individual slot can extend more than halfway around, if for example, increased flexibility is important. Conversely, if it is desired to provide additional column strength with less flexibility, each individual slot may extend less than halfway around the hypotube. An assembly including a sleeve disposed about the hypotube is disclosed. The sleeve may be formed of any suitable polymeric material, like a shape memory material such as a shape memory polymer, in particular a thermoplastic copolyester (TPC).

US 2016/193810 A1 discloses an electrically activated shape memory polymer composite capable of thermal shape reformation, using electric power to heat the composite through its matrix glass transition temperature. The composite includes an adaptable polymer matrix component using a diglycidyl ether resin, at least one substantially well-dispersed conductive or magnetic nano-filler component, and at least one elastic, laminated layer. The laminated layer is a sheet such as a silicone elastomer, an elastic metal alloy, or a combination thereof. The elastic, laminated layer contributes to enhancing the thermo-mechanical characteristics of the composite. The nano-filler (such as reduced graphene oxide sheets, carbon nanotubes, and/or iron oxides) is dispersed throughout the polymer matrix, the matrix having at least one external side contacting the laminated layer.

WO 2016/126703 A1 discloses a thermoset polymer with shape memory properties obtained by crosslinking an allyl-functionalised PU with a polythiol compound, preferably a mercaptoester. One embodiment includes crosslinkable alkene groups substantially regularly spaced along a polymer chain and a polythiol crosslinker.

Further reduction in the diameter of catheters based on technologies using materials that are potentially toxic and must be encapsulated in a cavity, for example a cavity provided in a double walled catheter are intrinsically limited. However, there is a need for further reduction in the diameter of catheters to further improve minimally invasive surgery.

The object of the present invention is to use a variable stiffness material in a thread-like variable stiffness device to allow the variation of the device stiffness, and to provide a thread-like variable stiffness device, particularly a catheter, which diameter can be further reduced.

The problem is solved by a thread-like variable stiffness device according to claim 1. Further preferred embodiments are subject of the dependent claims.

The device according to the present invention is a thread-like variable stiffness device comprising an elongate shaft, said shaft comprising a polymeric material, and a heat conducting device configured to exchange heat with the polymeric material to cause a phase transition of the polymeric material conferring a variable stiffness to the thread-like device.

Said device is characterized in that the polymeric material is a thermoset shape memory material. Due to the interaction of the thermoset shape memory material and the heat conducting device it is possible to provide a device with multiple variable stiffness segments.

Within the context of the present invention the term "thermoset shape memory material" refers to a thermoset (covalently crosslinked) polymer having a glass transition temperature such that when the polymer is brought to a temperature above its glass transition temperature, it can be deformed into a metastable state and then cooled below said transition temperature, at which point that metastable state can be fixed. Reheating above the transition temperature allows shape recovery at a force proportional to the rubbery modulus. The crosslinking can be carried out for example through UV light or heat. In addition, the thermoset shape memory material does not melt because the chemical crosslinking introduces stable net points in the polymeric matrix providing a strong microstructure. Thus, there is no need to have an additional layer to protect or encapsulate the thermoset polymer, since no material leakage is possible.

Further, according to the invention, the thermoset shape memory material is a mercaptoester polyurethane.

Further thermoset shape memory materials can be selected from the group consisting of a crosslinked poly(DL-lactic acid), a crosslinked poly(d,l-lactide-co-trimethylene carbonate), a crosslinked poly(L-lactide), a crosslinked poly(lactide-co-glycolide), a crosslinked blend of poly(ε-caprolactone) and a poly (propylene carbonate), a crosslinked polyurethane, a crosslinked poly(urea-urethane), epoxy resins, acrylic-based and methacrylic-based resins and their copolymers. Said polymers are biocompatible and provide good mechanical properties.

The thermoset shape memory material mercaptoester polyurethane, as well as thermoset shape memory material selected from the group consisting of a crosslinked poly(DL-lactic acid), a crosslinked poly(d,l-lactide-co-trimethylene carbonate), a crosslinked poly(L-lactide), a crosslinked poly(lactide-co-glycolide), a crosslinked blend of poly(ε-caprolactone) and a poly (propylene carbonate), a crosslinked polyurethane, a crosslinked poly(urea-urethane), epoxy resins, acrylic-based and methacrylic-based resins and their copolymers have a phase transition temperature in the range of 10 to 60°C, preferably in the range of 20 and 47°C. Such a phase transition temperature is especially preferred for medical applications since this allows to provide a flexibility when inserted into the body and thus to protect blood vessels or tissue, on the other hand, it provides the required stiffness to prevent a coiling up because of the frictional forces. The glass transition temperature (Tg) can be tuned, for example by varying the crosslinking processes (such as temperature and/or UV dose and/or exposure time) .

Within the context of the present invention the expression mercaptoester polyurethane stands for a polymer comprising a urethane component and a mercaptoester component. Examples of such mercaptoester polyurethanes are the Norland Optical Adhesives (NOA) marketed by Norland Products Inc.

The expression crosslinked poly(d,l-lactic acid) (PLA) stands for an aliphatic polyester produced from the monomer lactic acid, which is at least partly modified with functional groups. Thus, at least a part of the monomers or oligomers of lactic acid are chemically functionalized with functional groups in order to obtain a crosslinked polymer. Said functional groups are preferably selected from the group consisting of acrylate, methacrylate, epoxy, acrylamide, methacrylamide and vinyl groups or a mixture thereof. An example for such a oligomer modified with functional groups is a poly(lactic acid) telechelic oligmer endcapped with acrylate groups having a molecular weight from 700 to 10'000 g/mol.

The expression crosslinked poly(d,l-lactide-co-trimethylene carbonate stands for a copolymer consisting of polylactide and poly(trimethylene carbonate). The lactide ratios can vary between 40% to 70%. The poly(lactide-co-trimethylene carbonate) copolymers can be prepared for example by ring opening polymerization. Trimethylene carbonate is a suitable comonomer to modify PLA for biomedical uses, since it can impart weaker acidity and higher flexibility to the polymeric chains. At least a part of the monomers or oligomers of lactic acid and/or trimethylene carbonate are chemically functionalized with functional groups in order to obtain a crosslinked polymer. Said functional groups are preferably selected from the group consisting of acrylate, methacrylate, epoxy, acrylamide, methacrylamide and vinyl groups or a mixture thereof. The glass transition temperature is between 30 and 70°C and essentially depends on the ratio of TMC and PLA and on the crosslinking rate of the functional groups.

The expression poly(L-lactide) stands for a synthetic, aliphatic polyester produced from the monomer L-lactic acid which is at least partly modified with functional groups. Thus, at least a part of the monomers of l-lactic acid are chemically functionalized with functional groups in order to obtain a crosslinked polymer. Said functional groups are preferably selected from the group consisting of acrylate, methacrylate, epoxy, acrylamide, methacrylamide and vinyl groups or a mixture thereof.

The expression Poly(d,l-lactic acid-co-glycolic acid) is a copolymer composed of 2-hydroxypropanoyl and 2-hydroxyacetyl units. It is a copolymer macromolecule and a polyester macromolecule. At least a part of the monomers or oligomers of lactic acid and/or of glycolic acid are chemically functionalized with functional groups in order to obtain a crosslinked polymer. Said functional groups are preferably selected from the group consisting of acrylate, methacrylate, epoxy, acrylamide, methacrylamide and vinyl groups or a mixture thereof.

The expression a crosslinked blend of poly(ε-caprolactone) and a poly(propylene carbonate) stands for a copolymer of poly (ε-caprolactone) and poly(propylene carbonate with a weight ratio range between 90:10 and 10:90. At least a part of the monomers or oligomers of propylene carbonate are chemically functionalized with functional groups in order to obtain a crosslinked polymer. Said functional groups are preferably selected from the group consisting of acrylate, methacrylate, epoxy, acrylamide, methacrylamide and vinyl groups or a mixture thereof.

The term "crosslinked polyurethane" stands for polymers obtained by polymerization aliphatic isocyantes and diols, in particular multi-arm diols. The isocyanate composition used to prepare the crosslinked polyurethane can comprise any of the aliphatic, aromatic or araliphatic isocyanates or any of their mixtures, as well known in the art. The isocyanate composition may also comprise so-called modified isocyanates, obtained by introducing uretonimine, carbodiimide-, allophanate- , biuret- etc. bonds into the isocyanates. So called isocyanate-terminated prepolymers, which are prepared by pre-reacting the isocyanate composition with part of the isocyanate-reactive composition can also be used. Preferred isocyanates are aromatic isocyanates and isomers, homologs, variants and prepolymers thereof, especially preferred are diphenylmethane diisocyanate (MDI) and toluene diisocyanate (TDI) and their mixtures, modified variants and prepolymers. Most preferred are MDI- based isocyanates. All these compounds are well known in the art and commercially available.

Within the context of the present invention the expression a crosslinked poly(urea-urethane) refers to polymers that include both urea and urethane functional groups und is a preferred subgroup of the above-mentioned polyurethanes.

The expression epoxy resins refers to polymers which contain epoxide groups.

The term "acrylic-based resins" stands for polymers obtained by polymerization of functional acrylate monomers, preferably selected from the group consisting of 2-hydroxyethyl acrylate, methyl 2- cyanoacryalate, 2- and acrylic acid.

The term "methacrylic-based resins" stands for polymers obtained by polymerization of functional acrylate monomers, preferably selected from the group consisting of 2-hydroxyethyl methacrylate, 2-hydroxypropyl methacrylate, methacrylic acid, dimethylaminoethylmethacrylate and isocyanatoethyl methacrylate.

The thermoset shape memory material of the device according to the present invention comprises or is essentially made of a mercaptoester polyurethane comprising at least urethane or thiourethane linkages, that is carbamate or thiocarbonate functional groups, and a mercaptoester functional group. Said mercaptoester polyurethane is preferably formed via a thiole-ene reaction triggered by a photoinitiator, for example by reacting at least a component A comprising a compound having at least two alkene groups, preferably acrylate or methacrylate groups, and a component B comprising a compound having at least two thiol groups. The urethane linkage may be incorporated by reaction of a separate component C with a functional group of component A and/or component B or it may be an integral part of component A and/or component B. Most preferably, said mercaptoester is formed by a multifunctional ene- monomer as component A and a multifunctional thiol as component B.

Within the context of the present invention a multifunctional ene-monomer stands for a compound comprising three or more alkene groups.

Within the context of the present invention a multifunctional thiol stands for a compound comprising three or more thiol groups. Such compounds are also known as multi-arm thiols.

Component A of the mercaptoester polyurethane is preferably selected from the group consisting of triallyl cyanurate, triallyl thiocyanurate, trimethallyl thiocyanurate, 2-hydroxyethyl cyanurate tris(acrylate), 2-hydroxyethyl cyanurate tris(methacrylate), 2-hydroxyethyl cyanurate tris(allyl carbonate), 2-hydroxyethyl cyanurate tris(methallyl carbonate), triallyl isocyanurate, triallyl isothiocyanurate, trimethallyl isothiocyanurate, 2-hydroxyethyl isocyanurate tris(acrylate), 2-hydroxyethyl isocyanurate tris(methacrylate), 2-hydroxyethyl isocyanurate tris(allyl carbonate) and 2-hydroxyethyl isocyanurate tris(methallyl carbonate).

Component B of the mercaptoester polyurethane is preferably selected from the group consisting of pentaerythritol tetrakis (3-mercaptopropionate), ethoxylated pentaerythritol tetrakis(3-mercaptopropionate), thiol-functionalized polydimethyl siloxanes, thiol-terminated polysulfides, dipentaerythritol hexakis thioglycolate, trimethylolpropane tris(2-mercaptoacetate), pentaerythritol tetrakis(2-mercaptoacetate), tripentaerythritol octakis thioglycollate, mercaptan-terminated propoxylated glycerol, ethyleneglycol bis(3-mercaptopropionate) and trimethylolpropane tris(thioglycolate.

Especially good results could be obtained by a mercaptoester polyurethane marketed under the reference NOA 63 or by NOA 86 by Norland Products Inc. NOA 63 comprises a tetrathiol, triallyl isocyanurate and a polyurethane. NOA 86 comprises pentaerythritol and an acrylic monomer.

Preferably, the thermoset shape memory material comprises an electrically conductive filler which allows the flow of a current through the thermoset shape memory material. This allows to significantly reduce the final diameter of the device according to the present invention, which is an important prerequisite to produce minimally invasive surgical devices. Preferably, said electrical filler is selected from the group consisting of carbon based material, powered metals and mixture thereof. Examples of powdered metals are neodymium, iron, copper, cobalt and samarium and alloys thereof. Example of carbon based materials are graphene, graphite fibers and carbon nanotubes. In addition, it was shown that the presence of said electrically conductive filler can positively influence the mechanical properties and the glass transition temperature of the thermoset shape memory material. For example, it could be shown that an increase in graphene concentration slightly decreases the glass transition temperature.

Especially good results could be obtained by neodymium and graphene particles. Neodymium, besides electrical conductivity, has also magnetic properties which could be useful to steer the device. The amount of filler which is preferably present in the thermoset shape memory material depends on the filler and on its particle size.

The thread-like variable stiffness device according to the invention can be designed as medical instrument, more specifically as an endoscope or as a small-diameter continuum surgical instrument like a catheter, navigable within the body of a patient. It can be used in medical applications like diagnostic, therapeutic, mapping and ablative procedures, to name some examples.

The thread-like variable stiffness device can be designed to have an at least approximately rotationally symmetrical structure in relation to its longitudinal axis. The elongate shaft can have a long, slender, at least approximately cylindrical form, whose longitudinal axis can correspond to the longitudinal axis of the thread-like variable stiffness device. It has a proximal end, next to or nearest to a point of control of the device by an operator, and a distal end, situated away from the point of control and closest to an operation site. The elongate shaft comprises a distal end region arranged at its distal end.

The thermoset shape memory material used in the thread-like variable stiffness device according to the invention does not melt and does not become liquid like known low melting point alloy used in thread-like variable stiffness devices cited above. Indeed, it is in a transition state above the glass transition temperature, and in a rigid glassy state below the glass transition temperature. Consequently, the thermoset shape memory material does not need to be encapsulated in the shaft, i.e. no encapsulation layer is needed.

This allows a simplification of the structure of the thread-like variable stiffness device, so that the final diameter of the whole device can be significantly reduced, and the fabrication of a minimally invasive device is possible.

The heat conducting device is designed to exchange heat with the thermoset shape memory material, i.e. to heat or to cool the thermoset shape memory material, to increase or decrease its temperature in a controlled manner with respect to its glass transition temperature. Starting from a temperature of the thermoset shape memory material below the glass transition temperature at which it is in a rigid glassy state, the temperature can be increased in a controlled manner above the glass transition temperature to obtain a phase change in which the thermoset shape memory material is in rubber state. By reducing thereafter its temperature below the glass transition temperature, the thermoset shape memory material returns to its rigid glassy state.

As result, the thermoset shape memory material undergoes a phase transition and the stiffness of the device can be varied depending on the temperature of the thermoset shape memory material.

Concretely, the heat conducting device can comprise a heating loop designed to heat the thermoset shape memory material and a cooling loop designed to cool the thermoset shape memory material. The heating loop can include for example an electrical resistance embedded in the shaft, said heating loop comprising a metal wire or a conductive polymer. The cooling loop can comprise a channel for delivering a cooling fluid in the vicinity of the thermoset shape memory material.

Depending on the glass transition temperature of the thermoset shape memory material and the temperature at which the thread-like variable stiffness device is used, it is conceivable that the heat conducting device comprises only a heating loop and the cooling function is provided by the environment of the thread-like variable stiffness device. Similarly, it is conceivable that the heat conducting device comprises only a cooling loop and the heating function is provided by the environment of the thread-like variable stiffness device.

The temperature of the thermoset shape memory material is monitored by a temperature sensor connected to a control unit of the heat conducting device. The temperature sensor can be designed as a control coil comprising a conductive wire, which resistivity variation upon temperature is known, centered on the longitudinal axis of the shaft. The temperature sensor is preferably at least partially embedded in the thermoset shape memory material to improve its temperature monitoring.

In the present invention, the shaft comprises a thermoset material body made of the thermoset shape memory material and extending along the longitudinal axis of the shaft, and a heat conducting member of the heat conducting device, said heat conducting member extending, preferably longitudinally, over at least a portion of the thermoset material body and being designed to exchange heat with the thermoset material body.

The extension of the heat conducting member over at least a portion of the thermoset material body allows the heat exchange with the thermoset shape memory material and therefore a variation of its stiffness over at least their overlap length. As a result, the shaft becomes flexible or rigid over at least their overlap length depending on the temperature of the thermoset shape memory material.

A shaft segment over which the thermoset material body and the heat conducting member overlap to exchange heat defines a variable stiffness shaft segment.

In a preferred embodiment, the thread-like variable stiffness device can comprise a plurality of variable stiffness shaft segments extending one after the other in the longitudinal direction. Each variable stiffness shaft segment comprises a heat conducting member which can be controlled independently from each other, so that their respective stiffness can be controlled independently. This embodiment allows the navigation of the thread-like variable stiffness device in particularly tortuous passages because the stiffness of the variable stiffness shaft segments can be controlled and adjusted in one or several segments at each movement steps to adjust the bending possibilities to the passages. In this embodiment, it is possible to sequentially shape each variable stiffness shaft segments. By keeping a segment or several segments rigid, it is possible to obtain complex shape of thread-like variable stiffness device. This is particularly useful in organs with open volumes such as the hearth or stomach.

In a preferred embodiment, the variable stiffness shaft segments are connected directly one after the other in the longitudinal direction forming a continuous sequence of variable stiffness shaft segments. In other words, there are no rigid shaft segments between two variable stiffness shaft segments. This arrangement optimizes the bending of the thread-like variable stiffness device by approximating a continuous bending.

Within the context of the present invention, the term "rigid shaft" stands for a shaft segment which stiffness is not meant to be varied. Depending on the properties of the material of which the rigid shaft is made, the rigid shaft can naturally also be also at least partially flexible.

However, the provision of rigid shafts segments between two variable stiffness shaft segments is also possible. For example, this arrangement can be used to limit the bending radius of the device.

In a preferred embodiment, the heat conducting member is at least partially, preferably completely embedded in the thermoset material body. This allows a further reduction of the final diameter of the thread-like variable stiffness device compared to other arrangements, in which the heat conducting member must be arranged outside of the variable stiffness material, for example when the variable stiffness material is an encapsulated low melting point alloy. Indeed, the provision of a heat conducting member in an encapsulated low melting point alloy is subject to increased accidental rupture of the encapsulation and leakage of toxic substances in the body. This can happen for example at the connection or entry point of the heat conducting member in the encapsulation. With the reduction of the diameter, the fabrication of a minimally invasive device is improved. In addition, the provision of the heat conducting member in the thermoset shape memory material improves the temperature control of the thermoset shape memory material and consequently, the control of the stiffness changes.

In a preferred embodiment, the thread-like variable stiffness device is made essentially of a biocompatible thermoset shape memory material so that an outermost layer made of a biocompatible material is not necessary. This arrangement allows a further reduction of the outer diameter of the thread-like variable stiffness device and a particularly simple and economical manufacturing.

However, it is also conceivable to provide for an outermost biocompatible layer of the thread-like variable stiffness device to allow a medical use. This outermost layer can also be designed as a protective layer and/or as a thermal insulation layer in case the glass transition temperature is above a limit temperature defined by the environment in which the thread-like variable stiffness device is used. For example, the limit temperature at the outermost surface of the thread-like variable stiffness device should not be higher than approximately 45°C for a medical application in the human body, whereas the glass transition temperature of the thermoset shape memory material can be higher than 45°C. In this case, the provision of the thermal insulation layer allows the use thread-like variable stiffness device in the human body for a longer time than without insulation layer.

In a preferred embodiment, the shaft includes a conduit extending therethrough from the proximal end to the distal end of the shaft for the passage of a tool or a fluid. The provision of a conduit allows the adaptation of the thread-like variable stiffness device to different uses, depending on the tool introduced or provided in the conduit as well as the injection of therapeutic agents.

In a preferred embodiment, an information transfer device is arranged in the conduit to transmit information between the proximal end and the distal end of the shaft. Preferably, the information transfer device is an optic fiber or a signal wire connected to a tip device preferably arranged in the distal end region. The tip device can be a device used for medical application like diagnostic, therapeutic, mapping and ablative procedures, to name some examples. In particular, the tip device can be a sensor or a camera.

In the present invention, the thread-like variable stiffness device comprises a magnet arranged at its distal end to provide a means to control the distal end of the device, when it is submitted to a magnetic field. This feature makes it possible to achieve a greater precision of movement of the thread-like variable stiffness device. It is also conceivable to provide for multiple magnets arranged along the length of the thread-like variable stiffness device to allow for a more precise control of its shape.

In a preferred embodiment, the thermoset material body is configured as a tubular layer extending along a longitudinal axis of the shaft. Preferably, the tubular layer is at least approximately centered on the longitudinal axis of the shaft. The provision of the tubular layer allows a simple fabrication of the thread-like variable stiffness device, in particular if the tubular layer is centered on the longitudinal axis.

In a further preferred embodiment, the tubular layer has a ring-shaped circular cross section, preferably of a constant diameter over its length. The cylindrical symmetry of the arrangement obtained allows an easier control of the stiffness of the thread-like variable stiffness device.

In a preferred embodiment, the conduit is surrounded by the thermoset material body in the variable stiffness shaft segment, when viewed in cross section. In addition, the tubular structure allows, if necessary, the provision of the conduit centered on the longitudinal axis, so that the cylindrical symmetry and its advantages can be preserved.

In a preferred embodiment, the thermoset material body is configured as a solid tube, preferably having a circular cross section, extending along a longitudinal axis of the shaft. The provision of the solid tube allows a simplification of the fabrication of the thread-like variable stiffness device, in particular if it is centered on the longitudinal axis. Like in the previous embodiment, the cylindrical symmetry of this arrangement allows an easier control of the stiffness of the thread-like variable stiffness device.

In a preferred embodiment, the thread-like variable stiffness device has an outer diameter of less than 5 mm, preferably between 0,4 mm and 2,5 mm, to allow minimal invasive surgery for example in the heart region. An outer diameter between 0,4 mm and 1 mm is preferred to allow minimal invasive surgery in neurovascular applications.

In a preferred embodiment, the thread-like variable stiffness device is designed as a catheter having the features according to one or several of the embodiments disclosed above.

In a preferred embodiment, the thermoset material body is made of the thermoset shape memory material comprising, as disclosed above, an electrically conductive filler or a mixture of fillers containing at least an electrically conductive filler, and the thermoset shape memory material itself is the medium in which electric current can flow. Further, the heat conducting member comprises a first electrode and a second electrode, both embedded in the thermoset material body, arranged across the thermoset material body and configured to allow the passage of an electric current between them in the thermoset material body. As a result of the current flow between the first electrode and the second electrode, the thermoset shape memory material is heated by Joule effect. Consequently, in this embodiment, the provision of a heating member in the form of a wire or a coil is not necessary anymore and the final diameter of the thread-like variable stiffness device can be accordingly further reduced. Alternatively, for a given outer diameter, an increased volume of thermoset shape memory material can be provided in the thread-like variable stiffness device because the volume of the wire or the coil becomes available for thermoset shape memory material. Therefore, an increased stiffness variation is possible.

In a preferred embodiment, the first electrode is arranged in a proximal end region and the second electrode is arranged in a distal end region of the thermoset material body, viewed in the longitudinal direction. This arrangement increases the longitudinal extension of the segment whose stiffness can be varied and allows an increased bending. Preferably, the first electrode and the second electrode have the form of a disc which can be oriented perpendicularly to the longitudinal axis to improve the current conductive properties. In case the thread-like variable stiffness device comprises a conduit, the first electrode and the second electrode can have the form of an annular ring centered on the longitudinal axis of the conduit.

It is also conceivable to arrange the first electrode and the second electrode spaced apart radially with respect to the longitudinal axis. In this embodiment, the first electrode and the second electrode extend longitudinally and the current flows essentially perpendicular to the longitudinal axis.

In a preferred embodiment, the shaft is made of thermoset shape memory material comprising an electrically conductive filler over substantially its whole length. Preferably, the shaft comprises a plurality of variable stiffness shaft segments extending one after the other in the longitudinal direction, each comprising a first electrode and a second electrode. This embodiment allows a simple fabrication of the thread-like variable stiffness device.

In a preferred embodiment, it is also possible to provide for an electrode at the distal end of the most distal variable stiffness shaft segment and a further electrode at the proximal end of the most proximal variable stiffness shaft segment as well as each time a single intermediate electrode between each variable stiffness shaft segments to further simplify the fabrication.

Depending on the flexibility and retention needed for the application, for example a medical procedure, the variable stiffness properties can be adjusted by modifying structural parameters like the shape of the thermoset material body, the ratio between the area of the cross section occupied by the thermoset shape memory material to the total area of the cross section of the thread-like variable stiffness device, or the extension along the longitudinal axis of the thermoset material body, to name a couple of parameters. As an additional parameter, it is also conceivable to provide thermoset material bodies made each time of a different a thermoset shape memory material, wherein the thermoset material bodies are arranged at different positions in the thread-like variable stiffness device and have a different variable stiffness for a given temperature.

### Description of the figures

Fig. 1 shows a schematic perspective of a thread-like variable stiffness device according to the invention;
Fig. 2 shows a schematic cross-section along the line II-II of the thread-like variable stiffness device according to Fig. 1;
Fig. 3 shows a schematic cross-section of a further thread-like variable stiffness device according to an embodiment of the invention;
Fig. 4 shows a schematic partial cross-section side view of a distal end region of a further thread-like variable stiffness device according to the invention; and
Fig. 5 shows a schematic cross-section side view of a distal end region of a further thread-like variable stiffness device according to the invention.
Fig. 6 shows a schematic diagram of stiffness against temperature of an example of NOA.

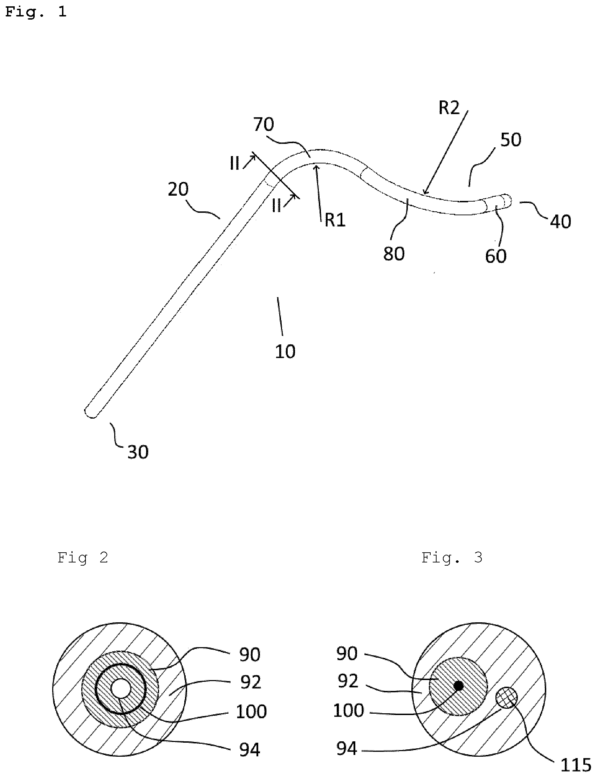

A thread-like variable stiffness device according to the invention is configured as a catheter 10 as illustrated in Fig. 1.

The catheter 10 has an at least approximately rotationally symmetrical structure in relation to its longitudinal axis with a narrow circular section in proportion to its length. It comprises an elongate shaft 20, whose longitudinal axis corresponds to the longitudinal axis of the catheter 10, having a circular section and extending over the whole length of the catheter 10. The catheter 10 has a proximal end 30 on the side of an operator, a distal end 40 on the side of the operation site and a distal end region 50 arranged at its distal end 40.

A magnet 60 is provided in the end region 50 to provide a means to control the distal end 40, when it is submitted to a magnetic field.

In the present embodiment, the catheter 10 comprises two variable stiffness shaft segments 70 and 80 extending directly one after the other in the longitudinal direction, i.e. without rigid shaft segments between them, and their stiffness can be controlled independently from each other, as illustrated by the different radius of curvature R1 and R2 of the variable stiffness shaft segments 70 and 80. For this purpose, as illustrated in Fig. 2, the variable stiffness shaft segments 70 and 80 comprise a thermoset material body 90 extending along the longitudinal direction of the variable stiffness shaft segments 70 and 80. The thermoset material body 90 has essentially a tubular form centered on the longitudinal axis of the variable stiffness shaft segment 70.

Further, the shaft 20 comprises a protective layer 92 surrounding the thermoset material body 90 and a conduit 94 extending inside the shaft 20 from the proximal end 30 to the distal end 40 for the passage of a tool, said conduit 94 being centered on the longitudinal axis of the shaft. However, it is also conceivable to provide for an outermost biocompatible layer of the catheter by choosing an adequate thermoset shape memory material. In this case, the protective layer 92, especially a biocompatible protective layer, is not necessary so that the outer diameter can be further reduced.

Further, the catheter 10 comprises a heat conducting device designed to exchange heat with the thermoset shape memory material in a controlled manner. In the embodiment of Fig. 2, the heat conducting device comprises each time a conducting member 100 in the respective variable stiffness shaft segments 70 and 80, said conducting member 100 extending along the longitudinal direction. Presently, the conducting member 100 is designed as a conductive coil 100 which allows heating by Joule effect of the thermoset shape memory material, when electric current passes through the coil 100. As result of the heat exchange, the thermoset shape memory material undergoes a phase transition when its temperature passes through the glass transition temperature and the stiffness of the device varies from stiff to flexible or from flexible to stiff depending on the direction of the temperature change.

In a further catheter embodiment illustrated in Fig. 3, the thermoset material body 90 is designed as a solid tube 90 having a circular section and extending parallel to the longitudinal direction. The catheter further comprises a heat conducting device having a conducting member in the form of a conductive wire 100 allowing heating by Joule effect of the thermoset shape memory material, when electric current passes through the conductive wire 100.

Further, the shaft 20 comprises a protective layer 92 surrounding the thermoset material body 90 and a conduit 94 extending inside the shaft 20. The conduit 94 extends parallel to the longitudinal direction therethrough from the proximal end to the distal end of the shaft for the passage of a tool. In the embodiment of Fig. 3 a tool 115 in the form of an optic fiber 115 is embedded in the protective layer 92 and schematically illustrated. It can be used to transfer information between the proximal end and the distal end, for example from a camera or a sensor located at the distal end and used for medical application like diagnostic or mapping.

In a similar embodiment not represented, a lumen extending inside the shaft 20 from a proximal end 30 to a distal end 40 of the shaft can be provided in the thermoset material body instead of the conductive wire 100. The lumen is part of a cooling loop designed to cool the thermoset shape memory material. In this case, a cooling fluid is delivered from the proximal end in the lumen to a variable stiffness shaft segment and returned to the proximal end.

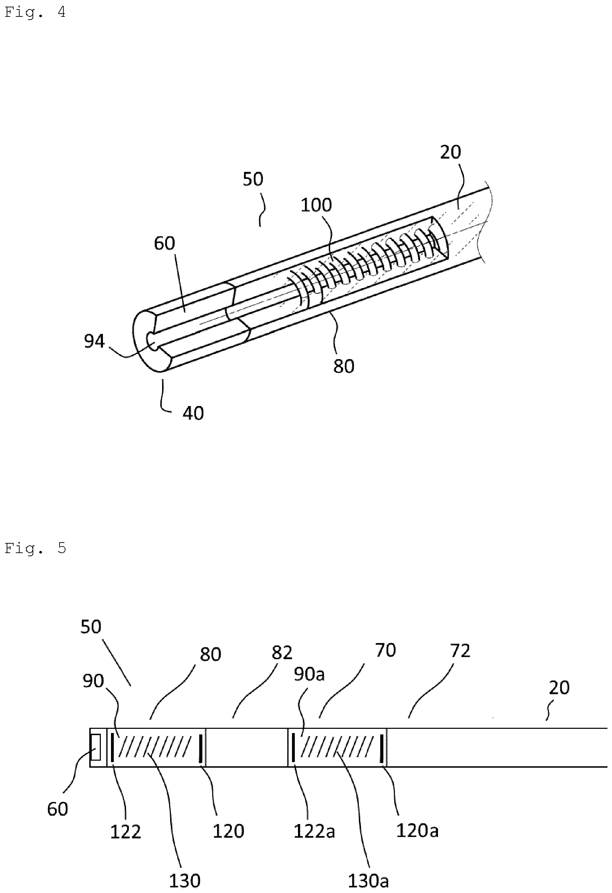

A further catheter according to the invention whose distal end region 50 only is illustrated in Fig. 4 has a particularly simple structure, allowing a simple and economical manufacturing. The catheter comprises a shaft 20 made essentially of biocompatible thermoset shape memory material so that no outermost layer made of a different material is required. In addition, the shaft includes a conduit 94 extending therethrough from the proximal end (no represented) to the distal end 40 of the shaft 20 for the passage of a tool, wherein the conduit 94 has a circular cross section and is centered on the longitudinal axis of the shaft. A permanent magnet 60 is arranged at the distal end 40 to provide means to control the distal end 40, when it is submitted to a magnetic field. The catheter further comprises a heat conducting device having a conducting member in the form of a conductive coil 100 in a variable stiffness shaft segment 80 located in the end region 50. The conductive coil 100 allows heating by Joule effect of the thermoset shape memory material, when electric current passes through the conductive coil 100.

The distal end region 50 of a further catheter 10 according to the invention is illustrated in Fig. 5. The catheter 10 is made of thermoset shape memory material comprising an electrically conductive filler comprising graphene and neodymium. Consequently, the flow of an electric current through the thermoset shape memory material is possible.

In the exemplary embodiment, the catheter 10 comprises at least a variable stiffness shaft segment 70 and a further variable stiffness shaft segment 80 extending one after the other in the longitudinal direction, viewed from the distal to the proximal end of the catheter, with a rigid shaft segment 82 arranged between the variable stiffness shaft segments 70 and the further variable stiffness shaft segment 80. A further rigid shaft segment 72 extends from the proximal end of the variable stiffness shaft segment 70 to the proximal end of the catheter.

The stiffness of the variable stiffness shaft segments 70 and 80 can be controlled independently from each other. In this embodiment, the rigid shaft segment 82 and the further rigid shaft segment 72 are also made of thermoset shape memory material comprising the electrically conductive filler. However, their stiffness is not varied during the use of the device.

The variable stiffness shaft segments 70 and the further variable stiffness shaft segment 80 comprise each time a thermoset material body 90a, 90 extending along the longitudinal direction and having substantially a tubular form centered on the longitudinal axis.

Further, the catheter comprises a heat conducting device designed to exchange heat with the thermoset shape memory material in a controlled manner. In the embodiment of Fig. 5, the heat conducting device comprises a heat conducting member arranged each time in the thermoset material body 90a, 90 of the variable stiffness shaft segments 70 and the further variable stiffness shaft segment 80, respectively.

Presently, each heat conducting member comprises a first electrode 120, 120a and a second electrode 122, 122a arranged across the thermoset material body 90, 90a respectively. The first electrode 120 is arranged in a proximal end region and the second electrode 122 is arranged in a distal end region of the further variable stiffness shaft segment 80, viewed in the longitudinal direction. The first electrode 120a and the second electrode 122a are arranged similarly in the variable stiffness shaft segment 70.

The first electrode and second electrode are configured to allow the passage of an electric current between them in the thermoset material body. For the sake of clarity, the connecting electrical wires extending each time from the first electrode and the second electrode to the proximal end of catheter are not represented in Fig. 5. As a result of the current flow between the first electrode and the second electrode in each thermoset material body 90, 90a, the thermoset shape memory material can be heated by Joule effect in the variable stiffness shaft segments 70 and the further variable stiffness shaft segment 80.

A permanent magnet 60 is arranged at the distal end 40 to provide a means to control the distal end 40, when it is submitted to a magnetic field.

The catheter further comprises a temperature sensor in the form of a control coil 130, 130a in each shaft segment to measure the temperature in each variable stiffness shaft segments 70, 80. The temperature measurement can rely on the resistivity change upon temperature change of the control coil.

### Experimental results

All samples are prepared according to the following recipe. The mixture of NOA86H and filler is prepared by mixing the two components with a stirrer and a sonicator tip. The mixture is injected into a mold and cured with UV-A light (10 min) and heat (oven at 100°C for 30 min). UV-A light is used to cure the surface and avoid chemical interactions with the mold surface while heat is used to cure the bulk material.

Table 1 shows the effect of graphene and NdFeB fillers on the Youngs modulus (above and below the Tg), the Tg, the thermal conductivity, and the electrical conductivity.

The effect of fillers on the glass transition temperature and the mechanical properties were characterized with DMA (Dynamic mechanical analysis) measurements on cylindrical shaft segments. The addition of graphene and NdFeB particles increases the mechanical strength both in the rigid (-10°C) and flexible state (100°C). NdFeB, compared to Graphene, leads to a higher increase in mechanical properties which leads to a decreased stiffness variation.

The glass transition temperature is decreasing with increasing filler concentration. The effect on the glass transition temperature is comparable for both fillers.

The thermal and electrical conductivity were measured on a cubic sample. The thermal conductivity is increasing with filler concentration (NdFeB and graphene), however the increase is approx. doubled with graphene compared to neodymium.

An effect on the electrical conductivity is visible only in graphene/NOA samples. A concentration of higher or equal to 3 w% of graphene allows for direct joule heating. No increase in electrical conductivity in the NdFeB/NOA samples could be observed due to oxidation on the NdFeB surface.

The magnetic properties (e.g. remanence) are increasing linearly with NdFeB concentration. Hysteresis curves were measured in a VSM (Vibrating-sample magnetometry) on cylindrical shaft segments.

**Table 1**

| | **NOA** | **NOA w9% C** | **NOA w40% NdFeB** |
|---|---|---|---|
| E solid [MPa] | 2864 | 4200 | 4452 |
| E soft [MPa] | 29 | 45 | 59 |
| Tg [°C] | 75 | 51 | 53 |
| Lambda [W/mK] | 0.176 | 0.399 | 0.261 |
| Sigma [Sm] | 0 | 1.05 | 0 |
| Remanence [Oe] | 0 | 0 | 27 |

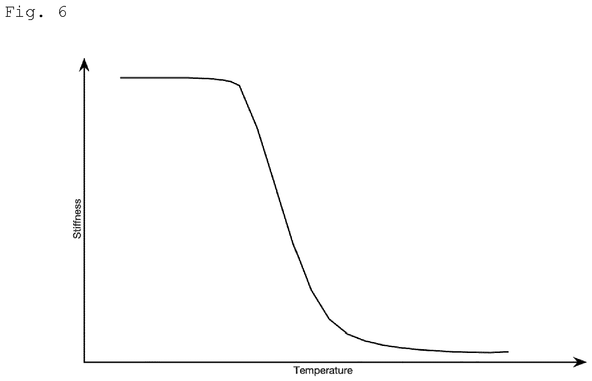

Figure 6 shows a diagram of stiffness against temperature of an example of NOA with or without filler. The curve can be characterized as follows. The first flat section relates to a temperature increase in the rigid state. The adjacent quick decrease in stiffness is related to the stiffness transition from a glassy to a rubbery state. The glass transition temperature is characterized as the point with maximum gradient. The curve flattens with increasing temperature in the rubbery state.

**List reference signs**

| | |
|---|---|
| thread-like variable stiffness device, catheter | 10 |
| elongate shaft | 20 |
| proximal end | 30 |
| distal end | 40 |
| distal end region | 50 |
| magnet | 60 |
| variable stiffness shaft segments | 70, 80 |
| thermoset material body | 90, 90a |
| radius of curvature | R1, R2 (of the shaft segments 70, 80) |
| protective layer | 92 |
| conduit | 94 |
| conductive coil | 100 |
| optic fiber | 115 |
| first electrode | 120, 120a |
| second electrode | 122, 122a |
| control coil | 130, 130a |

## Claims

1. A thread-like variable stiffness device (10) comprising an elongate shaft (20), said shaft (20) comprising a polymeric material, and a heat conducting device configured to exchange heat with the polymeric material to cause a phase transition of the polymeric material conferring a variable stiffness to the thread-like device, the polymeric material being a thermoset shape memory material, **characterized in that** the thermoset shape memory material is a mercaptoester polyurethane, and **in that** the shaft (20) comprises a thermoset material body (90, 90a) made of the thermoset shape memory material and extending along a longitudinal axis of the shaft, and a heat conducting member of the heat conducting device, said heat conducting member (130, 130a) extending along at least a portion of the thermoset material body and being designed to exchange heat with the thermoset material body, the device comprising a magnet (60) arranged at its distal end (40) to provide means to control the distal end (40) of the device, when it is submitted to a magnetic field.

2. Device according to claim 1, wherein the mercaptoester polyurethane is prepared at least by a component A comprising a compound having at least two alkene groups and a component B comprising a compound having at least two thiol groups.

3. Device according to claim 2, wherein component A comprises a compound selected from the group consisting of triallyl cyanurate, triallyl thiocyanurate, trimethallyl thiocyanurate, 2-hydroxyethyl cyanurate tris(acrylate), 2-hydroxyethyl cyanurate tris(methacrylate), 2-hydroxyethyl cyanurate tris(allyl carbonate), 2-hydroxyethyl cyanurate tris(methallyl carbonate), triallyl isocyanurate, triallyl isothiocyanurate, trimethallyl isothiocyanurate, 2-hydroxyethyl isocyanurate tris(acrylate), 2-hydroxyethyl isocyanurate tris(methacrylate), 2-hydroxyethyl isocyanurate tris(allyl carbonate) and 2-hydroxyethyl isocyanurate tris(methallyl carbonate).

4. Device according to claim 2 or 3, wherein component B comprises a compound selected from the group consisting of pentaerythritol tetrakis (3-mercaptopropionate), ethoxylated pentaerythritol tetrakis(3-mercaptopropionate), thiol-functionalized polydimethyl siloxanes, thiol-terminated polysulfides, dipentaerythritol hexakis thioglycolate, trimethylolpropane tris(2-mercaptoacetate), pentaerythritol tetrakis(2-mercaptoacetate), tripentaerythritol octakis thioglycollate, mercaptan-terminated propoxylated glycerol, ethyleneglycol bis(3-mercaptopropionate) and trimethylolpropane tris(thioglycolate).

5. Device according to any of the preceding claims, wherein the thermoset shape memory material additionally comprises an electrically conductive filler.

6. Device according to any of the preceding claims, **characterized in that** the heat conducting member (130, 130a) is at least partially embedded in the thermoset material body (90, 90a).

7. Device according to claims 5 or 6, **characterized in that** the shaft includes a conduit (94) extending therethrough from a proximal end (30) to the distal end (40) of the shaft (20) for the passage of a tool.

8. Device according to claim 7, **characterized in that** an information transfer device is arranged in the conduit (94) to transmit information between the proximal end (30) and the distal end (40) of the shaft (20).

9. Device according to any one of claims 6 to 8, **characterized in that** the thermoset material body (90, 90a) is designed as a solid tube extending along a longitudinal axis of the shaft.

10. Device according to any one of claims 6 to 9, **characterized in that** the thermoset material body is made of the thermoset shape memory material according to claim 5, and **in that** the heat conducting member comprises a first electrode (120, 120a) and a second electrode (122, 122a) arranged across the thermoset material body (90, 90a) and configured to allow the passage of an electric current between them in the thermoset material body.

11. Device according to claim 10, **characterized in that**, viewed in the longitudinal direction, the first electrode is arranged in a proximal end region and the second electrode is arranged in a distal end region (50) of the thermoset material body (90, 90a).

12. Use of a thermoset shape memory material for the preparation of a device according to any of claims 1 to 11.

## Patentansprüche

1. Fadenförmige Vorrichtung (10) mit variabler Steifigkeit, enthaltend einen länglichen Schaft (20), wobei der Schaft (20) ein polymeres Material enthält, und eine wärmeleitende Vorrichtung, die so konfiguriert ist, dass sie Wärme mit dem polymeren Material austauscht, um einen Phasenübergang des polymeren Materials zu bewirken, wodurch der fadenförmigen Vorrichtung eine variable Steifigkeit verliehen wird, wobei das polymere Material ein duroplastisches Formgedächtnismaterial ist, **dadurch gekennzeichnet, dass** das duroplastische Formgedächtnismaterial ein Mercaptoester-Polyurethan ist und dass der Schaft (20) einen duroplastischen Körper (90, 90a) enthält, der aus dem duroplastischen Formgedächtnismaterial hergestellt ist und sich entlang einer Längsachse des Schafts erstreckt, sowie ein wärmeleitendes Element der wärmeleitenden Vorrichtung, wobei sich das wärmeleitende Element (130, 130a) entlang mindestens eines Teils des duroplastischen Körpers erstreckt und so gestaltet ist, dass es Wärme mit dem duroplastischen Körper austauscht, wobei die Vorrichtung einen Magneten (60) enthält, der an ihrem distalen Ende (40) angeordnet ist, um ein Mittel zur Steuerung des distalen Endes (40) der Vorrichtung bereitzustellen, wenn sie einem Magnetfeld ausgesetzt ist.

2. Vorrichtung nach Anspruch 1, wobei das Mercaptoester-Polyurethan zumindest durch eine Komponente A, enthaltend eine Verbindung mit mindestens zwei AlkenGruppen, und eine Komponente B, enthaltend eine Verbindung mit mindestens zwei Thiol-Gruppen, hergestellt wird.

3. Vorrichtung nach Anspruch 2, wobei Komponente A eine Verbindung enthält, die aus der Gruppe bestehend aus Triallylcyanurat, Triallylthiocyanurat, Trimethallylthiocyanurat, 2-Hydroxyethylcyanurat-tris(acrylat), 2-Hydroxyethylcyanurat-tris(methacrylat), 2-Hydroxyethylcyanurat-tris(allylcarbonat), 2-Hydroxyethylcyanurat-tris(methallylcarbonat), Triallylisocyanurat, Triallylisothiocyanurat, Trimethallylisothiocyanurat, 2-Hydroxyethylisocyanurat-tris(acrylat), 2-Hydroxyethylisocyanurat-tris(methacrylat), 2-Hydroxyethylisocyanurat-tris(allylcarbonat) und 2-Hydroxyethylisocyanurat-tris(methallylcarbonat).

4. Vorrichtung nach Anspruch 2 oder 3, wobei Komponente B eine Verbindung enthält, die aus der Gruppe ausgewählt ist, die aus Pentaerythritoltetrakis(3-mercaptopropionat), ethoxyliertem Pentaerythritoltetrakis(3-mercaptopropionat), thiolfunktionalisierten Polydimethylsiloxanen, thiolterminierten Polysulfiden, Dipentaerythritolhexakisthioglykolat, Trimethylolpropan-tris(2-mercaptoacetat), Pentaerythritol-tetrakis(2-mercaptoacetat), Tripentaerythritol-octakis-thioglykolat, mercaptanterminiertes propoxyliertes Glycerin, Ethylenglycol-bis(3-mercaptopropionat) und Trimethylolpropan-tris(thioglykolat) besteht.

5. Vorrichtung nach einem der vorhergehenden Ansprüche, wobei das duroplastische Formgedächtnismaterial zusätzlich einen elektrisch leitfähigen Füllstoff enthält.

6. Vorrichtung nach einem der vorhergehenden Ansprüche, **dadurch gekennzeichnet, dass** das wärmeleitende Element (130, 130a) zumindest teilweise in den duroplastischen Körper (90, 90a) eingebettet ist.

7. Vorrichtung nach einem der Ansprüche 5 und 6, **dadurch gekennzeichnet, dass** der Schaft eine Leitung (94) umfasst, die sich durch diesen von einem proximalen Ende (30) zum distalen Ende (40) des Schafts (20) erstreckt, um ein Werkzeug hindurchzuführen.

8. Vorrichtung nach Anspruch 7, **dadurch gekennzeichnet, dass** eine Informationsübertragungsvorrichtung in der Leitung (94) angeordnet ist, um Informationen zwischen dem proximalen Ende (30) und dem distalen Ende (40) des Schafts (20) zu übertragen.

9. Vorrichtung nach einem der Ansprüche 6 bis 8, **dadurch gekennzeichnet, dass** der duroplastische Körper (90, 90a) als massives Rohr ausgestaltet ist, das sich entlang einer Längsachse des Schafts erstreckt.

10. Vorrichtung nach einem der Ansprüche 6 bis 9, **dadurch gekennzeichnet, dass** der duroplastische Körper aus dem duroplastischen Formgedächtnismaterial nach Anspruch 5 hergestellt ist und dass das wärmeleitende Element eine erste Elektrode (120, 120a) und eine zweite Elektrode (122, 122a) enthält, die über den duroplastischen Körper (90, 90a) angeordnet und so konfiguriert sind, dass sie den Durchgang eines elektrischen Stroms zwischen ihnen in dem duroplastischen Körper ermöglichen.

11. Vorrichtung nach Anspruch 10, **dadurch gekennzeichnet, dass** in Längsrichtung gesehen die erste Elektrode in einem proximalen Endbereich und die zweite Elektrode in einem distalen Endbereich (50) des duroplastischen Körpers (90, 90a) angeordnet ist.

12. Verwendung eines duroplastischen Formgedächtnismaterials zur Herstellung einer Vorrichtung gemäß einem der Ansprüche 1 bis 11.

## Revendications

1. Dispositif filiforme à rigidité variable (10) comprenant un tige allongée (20), ladite tige (20) comprenant un matériau polymère, et un dispositif conducteur de chaleur configuré pour échanger de la chaleur avec le matériau polymère afin de provoquer une transition de phase du matériau polymère conférant une rigidité variable au dispositif filiforme, le matériau polymère étant un matériau thermodurci à mémoire de forme, **caractérisé en ce que** le matériau thermodurci à mémoire de forme est un polyuréthane mercaptoester, et **en ce que** la tige (20) comprend un corps en matériau thermodurci (90, 90a) constitué du matériau thermodurci à mémoire de forme et s'étendant le long d'un axe longitudinal de la tige, et un élément conducteur de chaleur du dispositif conducteur de chaleur, ledit élément conducteur de chaleur (130, 130a) s'étendant le long d'au moins une partie du corps en matériau thermodurci et étant conçu pour échanger de la chaleur avec le corps en matériau thermodurci, le dispositif comprenant un aimant (60) disposé à son extrémité distale (40) pour fournir des moyens de contrôle de l'extrémité distale (40) du dispositif, lorsqu'il est soumis à un champ magnétique.

2. Dispositif selon la revendication 1, dans lequel le polyuréthane mercaptoester est préparé au moins par un composant A comprenant un composé ayant au moins deux groupes alcènes et un composant B comprenant un composé ayant au moins deux groupes thiols.

3. Dispositif selon la revendication 2, dans lequel le composant A comprend un composé choisi dans le groupe constitué par le cyanurate de triallyle, le thiocyanurate de triallyle, le thiocyanurate de triméthallyle, le cyanurate de 2-hydroxyéthyle tris(acrylate), le cyanurate de 2-hydroxyéthyle tris(méthacrylate), le cyanurate de 2-hydroxyéthyle tris(allyl carbonate), le cyanurate de 2-hydroxyéthyle tris(méthallyl carbonate), l'isocyanurate de triallyle, l'isothiocyanurate de triallyle, isothiocyanurate de triméthallyle, isocyanurate de 2-hydroxyéthyle, tris(acrylate), isocyanurate de 2-hydroxyéthyle, tris(méthacrylate), isocyanurate de 2-hydroxyéthyle, tris(carbonate d'allyle) et isocyanurate de 2-hydroxyéthyle, tris(carbonate de méthallyle).

4. Dispositif selon la revendication 2 ou 3, dans lequel le composant B comprend un composé choisi dans le groupe constitué par le tétrakis(3-mercaptopropionate) de pentaérythritol, le tétrakis(3-mercaptopropionate) de pentaérythritol éthoxylé, les polydiméthylsiloxanes à fonction thiol, les polysulfures à terminaison thiol, hexakis thioglycolate de dipentaérythritol, tris(2-mercaptoacétate) de triméthylolpropane, tétrakis(2-mercaptoacétate) de pentaérythritol, octakis thioglycollate de tripentaérythritol, glycérol propoxylé à terminaison mercaptan, éthylèneglycol bis(3-mercaptopropionate) et triméthylolpropane tris(thioglycolate).

5. Dispositif selon l'une quelconque des revendications précédentes, dans lequel le matériau thermodurci à mémoire de forme comprend en outre une substance de remplissage électriquement conductrice.

6. Dispositif selon l'une quelconque des revendications précédentes, **caractérisé en ce que** l'élément conducteur de chaleur (130, 130a) est au moins partiellement noyé dans le corps en matériau thermodurci (90, 90a).

7. Dispositif selon la revendication 5 ou 6, **caractérisé en ce que** la tige comprend un conduit (94) s'étendant à travers celle-ci depuis une extrémité proximale (30) jusqu'à l'extrémité distale (40) de la tige (20) pour le passage d'un outil.

8. Dispositif selon la revendication 7, **caractérisé en ce qu'**un dispositif de transfert d'informations est agencé dans le conduit (94) pour transmettre des informations entre l'extrémité proximale (30) et l'extrémité distale (40) de la tige (20).

9. Dispositif selon l'une quelconque des revendications 6 à 8, **caractérisé en ce que** le corps en matériau thermodurci (90, 90a) est conçu comme un tube plein s'étendant le long d'un axe longitudinal de la tige.

10. Dispositif selon l'une quelconque des revendications 6 à 9, **caractérisé en ce que** le corps en matériau thermodurci est réalisé en matériau thermodurci à mémoire de forme selon la revendication 5, et **en ce que** l'élément conducteur de chaleur comprend une première électrode (120, 120a) et une seconde électrode (122, 122a) disposées en travers du corps en matériau thermodurci (90, 90a) et configurées pour permettre le passage d'un un courant électrique entre elles dans le corps en matériau thermodurci.

11. Dispositif selon la revendication 10, **caractérisé en ce que**, vue dans la direction longitudinale, la première électrode est disposée dans une région d'extrémité proximale et la seconde électrode est disposée dans une région d'extrémité distale (50) du corps en matériau thermodurci (90, 90a).

12. Utilisation d'un matériau thermodurci à mémoire de forme pour la préparation d'un dispositif selon l'une quelconque des revendications 1 à 11.
